(19) Europäisches Patentamt
European Patent Office
Office européen des brevets

(11) Numéro de publication : **0 097 563**
**B1**

(12) # FASCICULE DE BREVET EUROPÉEN

(45) Date de publication du fascicule du brevet : **10.09.86**

(51) Int. Cl.⁴ : **C 01 F 17/00**, C 07 F 5/00

(21) Numéro de dépôt : **83401152.0**

(22) Date de dépôt : **07.06.83**

(54) **Procédé de préparation de dispersions colloidales de dioxyde cérique dans des liquides organiques.**

(30) Priorité : **11.06.82 US 387401**
**24.01.83 US 460694**

(43) Date de publication de la demande :
**04.01.84 Bulletin 84/01**

(45) Mention de la délivrance du brevet :
**10.09.86 Bulletin 86/37**

(84) Etats contractants désignés :
**AT BE CH DE FR GB IT LI NL SE**

(56) Documents cités :
**DE-C- 846 398**
**FR-A- 2 359 192**
**US-A- 3 518 287**
**Le dossier contient des informations techniques présentées postérieurement au dépôt de la demande et ne figurant pas dans le présent fascicule.**

(73) Titulaire : **RHONE-POULENC SPECIALITES CHIMIQUES**
**"Les Miroirs" 18, Avenue d'Alsace**
**F-92400 Courbevoie (FR)**

(72) Inventeur : **Gradeff, Peter S.**
**P.O.Box 278 Pottersville**
**New Jersey 08979 (US)**
Inventeur : **Charte, Vincent J.**
**881 Jamestown Road East Windsor**
**New Jersey 08520 (US)**
Inventeur : **Schreiber, Fred G.**
**100 Lawrence Avenue Highland Park**
**New Jersey 08904 (US)**
Inventeur : **Davison, John F.**
**7 Walnut Street Edison**
**New Jersey 08817 (US)**

(74) Mandataire : **Dutruc-Rosset, Marie-Claude et al**
**RHONE-POULENC INTERSERVICES Service Brevets Chimie 25, Quai Paul Doumer**
**F-92408 Courbevoie Cédex (FR)**

EP 0 097 563 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention a pour objet un nouveau procédé de préparation de dispersions colloïdales de dioxyde cérique dans des liquides organiques inertes.

Il est bien connu que les savons métalliques sont utilisés comme siccatifs dans les formulations de peintures et vernis, en vue d'accélérer le séchage des huiles insaturées telles que l'huile de lin ou les résines synthétiques insaturées telles que les résines alkydes. On pense que le cation du savon métallique catalyse activement les processus d'oxydation et de polymérisation et que l'anion de l'acide sert de support au métal conférant ainsi la solubilité dans l'huile, l'insolubilité dans l'eau et la compatibilité avec les autres composants de la peinture.

Il ressort du brevet GB-A-1 236 085 que, de toute évidence, il est avantageux d'un point de vue économique d'incorporer autant de métal que possible par unité d'acide à condition que le savon obtenu soit soluble dans l'huile. On y parvient en mettant en œuvre des savons « basiques » dans lesquels le rapport du métal à l'acide est supérieur au rapport stœchiométrique, par exemple :

$$2RCOOH + PbO \rightarrow (RCOO)_2Pb + H_2O\text{-rapport stœchiométrique}$$

$$2RCOOH + 2PbO \rightarrow RCOOPb \cdot O \cdot Pb\ OOC \cdot R + H_2O\text{-savon « basique »}$$

Cependant, le brevet indique que la solution de savon et d'huile que l'on obtient, lors de la préparation des savons « basiques » de ce type, est tellement visqueuse qu'elle est difficile à manipuler, en particulier au cours des opérations de mélange nécessaires durant la fabrication des compositions de peinture. Selon le brevet britannique, on peut réduire cette viscosité élevée en faisant réagir le milieu réactionnel de l'acide carboxylique ou de son sel de métal alcalin avec un sel de métal polyvalent ou un oxyde métallique apportant le cation métallique du siccatif.

Le sel de métal polyvalent ou l'oxyde métallique utilisé dans ledit procédé est un sel ou un oxyde d'aluminium, de baryum, de cuivre, de fer ou de magnésium ; de préférence de zirconium, de zinc ou de manganèse ; et encore plus préférentiellement de calcium, de plomb ou de cobalt. Il est recommandé de mélanger les différents savons métalliques, d'autant plus que certains savons tels que les savons de zinc et de calcium n'agissent pas eux-mêmes comme siccatifs, mais exercent un effet de synergie sur d'autres savons tels que les savons de cobalt ou de plomb. Il n'est pas fait référence aux savons de cérium ou des métaux de terres rares.

Le brevet GB-A-972 804 décrit des savons organiques de métaux contenant de l'aluminium ou du bore et au moins un métal divalent ou un radical métallique divalent (les atomes d'aluminium ou de bore et du métal divalent étant reliés par des atomes d'oxygène) et au moins un radical d'acide carboxylique. On obtient ces composés organiques de métaux par condensation des alcoxydes ou aryloxydes d'aluminium ou de bore avec des acyloxydes de métaux ou de radicaux métalliques divalents. Les métaux et les radicaux métalliques divalents comprennent le magnésium, le calcium, le strontium, le baryum, le zinc, le cadmium, le fer, le cobalt, le nickel, le plomb, le cuivre, le manganèse et le radical zirconyle, mais il n'est pas fait référence aux métaux ou radicaux des terres rares tel que le cérium. Les produits ont une teneur élevée en métal, les radicaux acides organiques étant présents dans la proportion de 0,5 à 1,5 équivalents par atome de métal. Il en résulte que les produits ont un potentiel d'acceptation des acides supérieur à celui des savons métalliques classiques. Ceux-ci constituent donc un exemple du type de savons « basiques » dont il est question dans le brevet GB-A-1 236 085 précité.

Il est mentionné dans le brevet GB-A-1 430 347 de Collins et Pearl que les savons métalliques normaux ou basiques d'acides carboxyliques synthétiques étaient des composés analogues à ceux qui dérivaient antérieurement d'acides naturels, et qu'ils ont renfermé des composés ayant entre eux une relation plus ou moins homologue sinon isomère lorsque l'on a utilisé les différents acides carboxyliques synthétiques au fur-et-à-mesure de leur disponibilité. Collins et al. proposent, à partir de cet art antérieur, de mettre en œuvre une méthode de préparation différente et une composition différente ce qui a pour résultat de conférer au produit siccatif ou savon métallique obtenu un caractère et des propriétés différents.

Dans l'art antérieur, selon Collins *et al.*, les savons métalliques ont été préparés par des procédés de fusion ou de précipitation. L'acide qui va réagir peut être dissous dans un solvant inerte approprié, généralement un solvant hydrocarboné, tel qu'une essence minérale, auquel on ajoute ensuite le composé métallique désiré, généralement sous forme d'un oxyde, d'un composé inorganique ou d'un sel approprié, en chauffant à une température appropriée pour favoriser la réaction. On obtient ainsi une solution hydrocarbonée de ce savon et le solvant peut être éliminé par distillation pour augmenter la concentration en métal à la valeur souhaitée.

Le procédé Collins et al. consiste à mettre en œuvre un acide carboxylique ou un mélange d'acides qui peuvent être d'origine naturelle, dérivés d'un produit naturel ou d'origine synthétique et à le mélanger avec un glycol-éther, un glycol ou autre polyol analogue, tout en ajoutant également un composé métallique tel que le métal sous forme de poudre ou un oxyde, hydroxyde, acétate ou carbonate dudit métal. On chauffe alors ce mélange à une température allant de 65° à 143 °C jusqu'à ce que le composé métallique disparaisse. Ensuite, on élimine l'eau par distillation, on filtre le mélange réactionnel et on

élimine l'excès de glycol ou de glycoléther par distillation jusqu'à obtention de la concentration ou de l'état convenable souhaité.

Le rapport des équivalents de métal au glycol-éther ou polyol est d'au moins 0,5, mais il faut conserver une quantité significative de glycol-éther ou de polyol dans le produit si l'on souhaite qu'il reste fluide. Le rapport des équivalents de métal à l'acide est d'au moins 1,0 et lorsque le métal est le plomb, il est d'au moins 1,5 ; avec le plomb on obtient facilement des rapports de 2 et plus. En dehors du plomb, on a préparé également selon ce procédé des savons de baryum, de nickel et de manganèse ainsi que des savons de cobalt. Cependant, on n'y trouve aucune référence aux métaux de terres rares tels que le cérium.

Il est précisé dans ce brevet que le produit et le procédé sont nettement différents des propositions de l'art antérieur, par exemple, l'emploi de quantités variables de glycol ou de glycol-éther simplement pour réduire la viscosité du carboxylate de plomb comme dans le brevet GB-A-1 148 998 ou pour stabiliser des solutions de savon comme dans le brevet US-A-2 807 553 de Fisher. Ces produits sont commercialisés par la Société Mooney Chemicals.

La demande de brevet FR-A-2 359 192 et le brevet GB-A-1 571 210 concernent des sels organiques de cérium solubles dans les solvants organiques, caractérisés par un rapport r du nombre d'équivalents d'acide au nombre d'atomes de cérium compris entre 0,2 et 1 ; le nombre d'équivalents d'acide représente le nombre de molécules d'acide lorsque l'acide utilisé est monofonctionnel, et il faut doubler ou tripler ce nombre, dans le cas de diacides ou triacides et, plus généralement, le multiplier par le nombre de fonctions acide dans le cas d'un polyacide. Les composés du cérium ainsi proposés nécessitent une quantité d'acide beaucoup plus faible que la quantité utilisée antérieurement pour obtenir la même efficacité ; on peut également obtenir des solutions de concentration élevée en métal pouvant aller jusqu'à 500 g/l ; les solutions obtenues restent fluides et peuvent être manipulées sans aucune difficulté tout en restant parfaitement solubles dans les milieux d'hydrocarbures.

L'acide organique peut être RCOOH, $RSO_3H$, $ROSO_3H$, $ROPO_3H_2$ ou $(RO)_2PO_2H$, R étant un radical hydrocarboné ayant au moins 7 atomes de carbone. Le radical acide organique peut être un radical aliphatique linéaire ou ramifié ou un radical cycloaliphatique éventuellement substitué par un radical alcoyle ou un radical aromatique éventuellement substitué par un radical alcoyle. Les sels de cérium de ces acides organiques peuvent, en plus, contenir au moins un autre métal de terres rares et ce, jusqu'à 25 % de la teneur totale en métaux de terres rares, y compris le cérium. On peut obtenir des compositions fournies sous la forme de solution dans un solvant organique, d'un sel de cérium d'acide organique ou d'un mélange de sels contenant plus de 200 g de cérium par litre de composition. Ces compositions peuvent être ajoutées aux peintures, vernis ou combustibles liquides.

Le procédé de préparation de ces sels de cérium d'acide organique ou de leurs mélanges consiste à faire réagir dans un milieu organique ou hydroorganique, l'acide organique et l'hydroxyde de cérium $Ce(OH)_3$ fraîchement préparé de sorte que les sels de cérium d'acide organique obtenus aient un rapport r compris entre 0,2 et 1. La réaction s'effectue de préférence en chauffant et le milieu organique est de préférence un hydrocarbure. Après plusieurs heures, une partie de l'eau qui s'est formée durant la réaction décante spontanément. Après réaction, il est possible d'aider à la séparation de l'eau formée à partir du milieu réactionnel en ajoutant un tiers solvant tel qu'un glycol, un alcool ou un alcoylglycol. On peut ajuster la concentration de la solution ainsi obtenue en ajoutant un hydrocarbure approprié.

Dans les exemples, l'hydroxyde de cérium $Ce(OH)_3$ est obtenu par précipitation du nitrate de cérium par l'ammoniaque. On lave le précipité à l'eau jusqu'à disparition de l'ion nitrate ; ensuite, on filtre le précipité jusqu'à ce qu'il ne contienne plus que 15 % d'eau. On fait réagir à 80 °C l'hydroxyde de cérium avec 130 g d'acide oléique technique dans du white-spirit. Après quatre heures on ajoute du glycol, on élimine l'eau qui s'est séparée et, ensuite, on ajoute du butyl-glycol, puis du white-spirit pour constituer la solution finale.

La demande de brevet FR-A-2 482 075 et le brevet US-A-4 356 106 sont relatifs à la préparation de dispersions aqueuses de composés du cérium qui peuvent être aisément dispersés. On obtient un produit dispersable en milieu aqueux en chauffant l'hydrate de dioxyde de cérium contenant des ions $NO_3^-$, $Cl^-$ ou $ClO_4^-$, pendant 1 à 2 heures, à des températures comprises entre 200 et 450 °C. Cependant, on ne donne aucune indication sur l'aptitude du produit à se disperser en milieu organique.

On notera que l'invention du brevet se rapporte aux sels céreux et non pas aux sels cériques.

Dans l'encyclopédie Kirk-Othmer, Encyclopedia of Chemical Technology (deuxième édition), volume 4, page 850, il est indiqué que l'oxyde cérique hydraté, également dénommé « hydrous ceric oxide » ou « cerium hydroxide » $CeO_2$ x $H_2O$, formule dans laquelle x est un nombre compris entre 1/2 et 2, est obtenu sous forme d'un précipité gélatineux lorsqu'on ajoute des hydroxydes de sodium ou d'ammonium aux solutions de sels cériques. Après séchage du précipité, on obtient un oxyde hydraté jaune contenant de 85 à 90 % de $CeO_2$. On peut obtenir de l'hydroxyde cérique granulaire en faisant bouillir des sels de cérium insolubles avec de l'hydroxyde de sodium concentré et en procédant ensuite à un lavage et un séchage. La composition et la structure de ces composés dépendent de leur mode de préparation ; dans de nombreux cas, elles sont incertaines. C'est la raison pour laquelle, dans la pratique, on exprime la composition en termes d'équivalent $CeO_2$.

L'hydroxyde céreux $Ce(OH)_3$ est obtenu sous forme d'un précipité gélatineux blanc ou blanchâtre lorsqu'on alcalinise des solutions contenant l'ion céreux $Ce^{3+}$. Quand on laisse reposer un certain temps,

il apparaît une couche superficielle d'hydroxyde céreux/cérique.

On obtient généralement l'oxyde cérique $CeO_2$ par calcination à l'air de l'oxalate céreux, de l'hydroxyde céreux ou cérique. L'oxyde cérique est insoluble dans les acides, mais sa dissolution est accélérée par l'addition d'une petite quantité d'agent réducteur tel qu'un iodure ou l'eau oxygénée. Eventuellement, les acides nitrique ou sulfurique concentrés réagissent à chaud.

Dans de nombreuses applications, on peut remplacer l'oxyde cérique par l'oxyde cérique hydraté. Cependant, à la différence de l'hydrolyse céreux qui est un type classique d'hydroxyde métallique analogue au $Pb(OH)_2$, au $Fe(OH)_3$ etc..., l'hydroxyde cérique est, en fait, du dioxyde cérique hydraté. Dans l'exposé qui suit de l'invention,. on entend par « dioxyde cérique » aussi bien le dioxyde cérique, l'hydroxyde cérique, le dioxyde cérique hydraté (quelle que soit la forme sous laquelle l'eau est retenue dans ou sur le produit : eau libre, eau liée ou adsorbée, eau de cristallisation).

Si l'on agite et chauffe du dioxyde cérique pur à une température comprise entre 60° et 200 °C en présence d'un solvant aliphatique, tel que l'éther de pétrole; ou d'un solvant aromatique, tel que le toluène, et en présence d'un acide carboxylique, tel que l'acide oléique ou palmitique ou dodécylbenzène sulfonique, il ne se produit aucune dispersion. Il n'y a pas davantage de réaction que ce soit avec l'acide carboxylique, alcoyl- ou alcoylarylsulfonique.

La présente invention a pour objet un nouveau type de dioxyde cérique colloïdal à forte teneur en cérium, qui est susceptible de se disperser dans des liquides organiques, notamment des solvants organiques. Elle concerne également des compositions à forte teneur en cérium contenant ledit dioxyde cérique colloïdal dispersé dans un liquide organique. Conformément à l'invention, les dispersions à forte teneur en cérium sont de vraies dispersions comme cela est démontré par microscopie électronique en transmission. L'expression « dioxyde de cérium dispersé » utilisée dans la description et les revendications signifie que les particules de dioxyde cérique sont de dimensions colloïdales et que, par conséquent, elles se trouvent sous la forme d'une dispersion colloïdale dans des liquides organiques, mais ceci n'exclut pas la présence de dioxyde cérique en solution, en plus ou à la place du dioxyde cérique dispersé sous forme colloïdale. L'examen au microscope électronique en transmission du dioxyde cérique hydraté avant traitement selon l'invention ne met pas en évidence de particules de dimensions colloïdales. La transformation dudit dioxyde cérique en particules de dimensions colloïdales se fait au cours du traitement.

La figure 1 représente une photographie prise au microscope électronique en transmission qui montre l'état particulaire cristallin d'un dioxyde cérique typique avant traitement effectué selon le procédé de l'invention.

La figure 2 représente une photographie prise au microscope électronique en transmission qui montre la forme particulaire du dioxyde cérique de la figure 1 après traitement effectué selon le procédé de l'invention.

Le procédé de l'invention qui permet de préparer des dispersions colloïdales de dioxyde cérique dans des liquides organiques inertes est caractérisé par le fait qu'il consiste :

1) à chauffer à une température comprise dans un intervalle allant d'environ 60 °C à environ 200 °C

a) du dioxyde cérique comprenant du nitrate d'ammonium en une quantité comprise entre environ 3 % et environ 14 % du poids du dioxyde cérique en présence d'eau, de méthanol, d'acide acétique ou de leurs mélanges en une quantité d'au moins 10 g par mole de $CeO_2$

b) un acide organique ayant environ 10 à environ 40 atomes de carbone soluble dans le liquide organique et mis en œuvre à raison d'au moins 0,25 mole par mole de $CeO_2$

c) un liquide organique choisi dans le groupe formé par les hydrocarbures aliphatiques, cycloaliphatiques et aromatiques ; les hydrocarbures chlorés ; les éthers aliphatiques et cycloaliphatiques ; les cétones aliphatiques et cycloaliphatiques formant ainsi une dispersion colloïdale dans le liquide organique du dioxyde cérique et de l'acide organique associé ;

2) à éliminer l'eau, le méthanol, l'acide acétique libéré pendant le chauffage et à séparer toutes les particules solides non dissoutes.

On obtient le dioxyde cérique colloïdal à partir de dioxyde cérique préparé spécialement pour servir de produit de départ dans le procédé de l'invention de telle sorte qu'il retienne par des associations physiques :

1) — de 3 environ à 14 % environ de nitrate d'ammonium

2) — de l'eau, du méthanol, de l'acide acétique ou les mélanges de deux ou trois quelconques des composants précités en une quantité d'au moins 10 g par mole de $CeO_2$.

1) et 2) sont essentiels et doivent obligatoirement être présents. On dénommera le produit obtenu « dioxyde cérique activé » ou « $CeO_2$ activé ».

Il a été mis en évidence expérimentalement que le procédé de l'invention peut être considéré comme une réaction physique adsorption-addition (par opposition à une réaction chimique substitution-élimination telle que la formation d'un sel) de l'acide organique, peut-être au niveau des interstices ou comme inclusion par chimisorption dans la structure du dioxyde cérique, qu'il soit cristallin ou non. Cette association physique se forme lors de la rupture de gros agglomérats de dioxyde cérique en cristallites ayant un diamètre d'environ 50 Å, par chauffage du dioxyde cérique activé précité, en présence d'un acide organique solubilisant ayant de 10 à 40 atomes de carbone et d'un liquide organique approprié, à une température variant dans l'intervalle d'environ 60 °C à environ 200 °C pendant une durée suffisante,

généralement de 1 heure à environ 24 heures, pour effectuer la désagrégation des agglomérats en cristallites de dimensions colloïdales et leur association avec l'acide solubilisant ; ensuite, on élimine l'eau, le méthanol ou l'acide acétique libéré, puis on élimine par filtration les sels qui se séparent lors du refroidissement.

Le complexe qui résulte de l'association physique $CeO_2$-acide peut être isolé de telles solutions colloïdales sous forme de particules colloïdales solides. La microscopie électronique en transmission des solutions colloïdales met en évidence des cristallites de 50 Å, parfaitement dispersées. Le complexe reste stable pendant un certain temps à condition qu'il soit conservé dans un récipient fermé. Si on le mélange avec un liquide organique approprié, on obtient aussitôt une dispersion colloïdale.

Le dioxyde cérique de départ peut être un dioxyde cérique anhydre ou un dioxyde cérique hydraté mais il est essentiel que le produit de départ du dioxyde cérique contienne de l'ordre de 3 à 14 % en poids de nitrate d'ammonium. On ne peut pas tout simplement mélanger ou ajouter le nitrate d'ammonium au dioxyde cérique. Il faut qu'il y ait une association physique étroite entre le nitrate d'ammonium et le dioxyde cérique ; il est possible que ce soit par inclusion du nitrate d'ammonium sous forme de molécule saline et/ou sous forme d'ions ammonium et nitrate dans la structure des agglomérats trouvés au cours de la préparation du dioxyde cérique hydraté. La deuxième condition exigée est la présence dans le système de la quantité précitée d'eau, de méthanol, d'acide acétique ou de leurs mélanges.

Un dioxyde cérique de départ convenant pour la préparation des produits de l'invention est commercialisé par la Société Rhône-Poulenc. On peut également le préparer par les procédés décrits dans les brevets, par exemple la demande de brevet française publiée sous le n° 2 482 075 qui décrit le traitement du nitrate céreux ou du carbonate céreux par de l'acide nitrique aqueux suivi par un traitement $NH_4OH$—$H_2O_2$. Aux fins de la présente invention,, le dioxyde cérique qui est récupéré, par exemple par filtration, centrifugation ou autre technique de séparation, n'a pas besoin d'être lavé ou s'il est lavé, il ne l'est pas suffisamment pour éliminer le nitrate d'ammonium occlus ; par conséquent, il contient par association physique de l'ordre de 3 à 14 % de nitrate d'ammonium résiduel ainsi qu'un peu de nitrate de cérium.

La quantité de nitrate peut varier en fonction des paramètres de procédé retenus pour la préparation, en fonction de la quantité des eaux-mères résiduelles ou de l'importance du lavage partiel lorsqu'il est effectué. Il va de soi que lorsque la base utilisée pour la précipitation est $NH_4OH$, les ions portés par le dioxyde cérique seront $NH_4^+$ et $NO_3^-$.

Le produit humide provenant directement du filtre contient donc une quantité variable d'eau. Dans le cas où la deuxième condition exigée est remplie par la présence d'eau, on peut noter qu'il faut au moins environ 10 g d'eau/mole de $CeO_2$ pour que le produit humide puisse être mis en œuvre dans le cadre de l'invention. Normalement, la quantité d'eau retenue dans dioxyde cérique hydraté qui vient d'être préparé est de l'ordre de 10 à 20 %. Il peut y avoir évidemment présence d'une plus grande quantité d'eau, mais c'est un inconvénient puisqu'il faudra l'éliminer plus tard au cours du procédé.

Il est surprenant de constater que si le méthanol peut se substituer à l'eau, les autres alcools inférieurs, tels que l'éthanol, restent sans effet et ne peuvent pas se substituer au méthanol.

De même, l'acide acétique est le seul acide que l'on puisse substituer à l'eau ou au méthanol ; on ne peut pas mettre en œuvre l'acide organique qui est utilisé pour la préparation du complexe obtenu par association physique. L'acide acétique aussi bien que l'eau ou le méthanol a, de toute évidence, une fonction particulière dans le mécanisme qui n'est pas encore entièrement élucidé de désagrégation des agglomérats en $CeO_2$ de dimensions colloïdales suivie par l'addition de l'acide solubilisant.

D'une manière préférentielle, on utilise l'eau, le méthanol ou l'acide acétique ou un mélange d'eau et de méthanol, d'eau et d'acide acétique ou d'eau, de méthanol et d'acide acétique.

La quantité d'eau, de méthanol, d'acide acétique ou de leurs mélanges est d'au moins 10 et de préférence inférieure à environ 60 g/mole de $CeO_2$.

Il ne faut pas procéder à un séchage prolongé du dioxyde cérique à des températures élevées, par exemple 375 °C ou plus, qui entraîneraient la décomposition du nitrate d'ammonium : dans ce cas, la teneur du dioxyde cérique obtenu en $NH_4NO_3$ pourrait tomber en dessous de la quantité minimum et ledit dioxyde cérique ne peut plus être utilisé dans le procédé de l'invention même après addition d'eau, de méthanol, d'acide ou même de nitrate d'ammonium libre.

Le milieu liquide organique utilisé dans le procédé de l'invention peut être un hydrocarbure aliphatique ou cycloaliphatique inerte ou leur mélange tel que par exemple des essences minérales ou de pétrole, éthers minéraux ou de pétrole pouvant contenir également des composantes aromatiques. Les exemples comprennent l'hexane, l'heptane, l'octane, le nonane, le décane, le cyclohexane, le cyclopentane, le cycloheptane et les naphtènes liquides. Les solvants aromatiques tels que le benzène, le toluène et les xylènes conviennent également.

On peut mettre en œuvre également des hydrocarbures chlorés tels que le chloro- ou dichlorobenzène et le chlorotoluène aussi bien que des éthers aliphatiques et cycloaliphatiques tels que l'éther de diisopropyle et les cétones aliphatiques et cycloaliphatiques.

On choisira le liquide organique ou système solvant en tenant compte de l'acide organique solubilisant utilisé, de la température de chauffage et de l'application finale de la solution ou dispersion colloïdale. Dans certains cas, il est préférable d'employer un mélange de solvants. La quantité de liquide ou solvant détermine évidemment la concentration finale. Les solutions contenant jusqu'à environ 50 %

de $CeO_2$ sont parfaitement fluides. Il est donc plus économique et plus commode de préparer des solutions plus concentrées qui pourront être diluées plus tard, lors de leur emploi. C'est pour cette raison que la quantité de solvant n'est pas critique.

L'acide organique servant à la formation du complexe par association physique est un acide organique ou un mélange d'acides qui est soluble dans le milieu solvant organique utilisé. On peut mettre en œuvre des acides carboxyliques aliphatiques, des acides sulfoniques aliphatiques, des acides phosphoniques aliphatiques, des acides alcoylarylsulfoniques et des acides alcoylarylphosphoniques possédant environ de 10 à environ 40 atomes de carbone, qu'ils soient naturels ou synthétiques. A titre d'exemples, on peut citer, les acides gras de tallöl, d'huile de coco, de soja, de suif, d'huile de lin, l'acide oléique, l'acide linoléique, l'acide stéarique, l'acide isostéarique, l'acide pelargonique, l'acide caprique, l'acide laurique, l'acide myristique, l'acide dodécylbenzènesulfonique, l'acide éthyl-2 hexoïque, l'acide naphténique, l'acide hexoïque, l'acide toluène-sulfonique, l'acide toluène-phosphonique, l'acide lauryl-sulfonique, l'acide lauryl-phosphonique, l'acide palmityl-sulfonique et l'acide palmityl-phosphonique.

Le type de l'agent solubilisant utilisé détermine souvent la quantité maximum de $CeO_2$ qui puisse être dissoute. Les acides alcoylarylsulfoniques sont aptes à donner des produits ayant de fortes concentrations en cérium.

L'acide organique est mis en œuvre à raison d'au moins 0,25 mole par mole de $CeO_2$ étant donné que le complexe obtenu par association physique $CeO_2$-acide présente un rapport $CeO_2$/acide organique de 4/1 comme le montre la composition de la forme solide isolée. On peut, certes, employer de plus petites quantités d'acide, mais on risque d'obtenir une dispersion incomplète du dioxyde cérique ou une dispersion relativement instable qui aura tendance à former un dépôt de $CeO_2$. On peut employer plus de 0,25 mole d'acide organique, mais ce n'est peut-être pas nécessaire.

La présence d'eau, de méthanol ou d'acide acétique ou de leur mélange est indispensable durant la réaction mais l'on n'explique pas très bien leur rôle. Tout au moins, on peut dire qu'ils contribuent à l'expulsion des ions nitrate d'une manière telle qu'il en résulte une désagrégation des agglomérats de $CeO_2$ en particules de dimensions colloïdales. La surface hautement activée des cristallites adsorbe alors l'acide qui les rend organodispersables. Si l'on enlève du système, l'un des activateurs volatils essentiels, à savoir l'eau, le méthanol ou l'acide acétique, avant que les processus désirés aient eu lieu, il peut arriver que la réaction ne se produise pas du tout ou soit incomplète.

Les dioxydes cériques hydratés techniques du commerce contiennent d'autres terres rares comme impuretés. Dans certains cas, on peut souhaiter la présence de telles impuretés en raison d'effets de synergie bénéfiques qu'elles peuvent présenter. On peut également utiliser selon le procédé de l'invention des mélanges de dioxyde cérique contenant jusqu'à environ 10 % d'autres terres rares.

La durée totale du chauffage est de moins d'une heure à environ 24 heures et plus et pendant ce temps, on agite et chauffe à une température comprise dans un intervalle allant de 60 °C environ à 200 °C environ.

En procédant à un chauffage préliminaire du dioxyde cérique de départ se présentant soit sous forme de bouillie aqueuse ou soit en mélange avec un liquide organique tel que les essences de pétrole, à une température comprise dans un intervalle allant de 60 °C environ à 200 °C environ, pendant plusieurs heures suivi de l'addition de l'acide organique utilisé pour former le complexe obtenu par association physique, tel que l'acide oléique, on obtient de manière significative des vitesses de solubilisation plus rapides. L'examen au microscope électronique du produit chauffé a révélé qu'il ne s'est produit aucune diminution de la taille des particules de dioxyde cérique et par conséquent, on est amené à penser que le chauffage affaiblit, mais ne rompt pas les ponts entre les cristallites de nitrate d'ammonium et/ou d'ions $NH_4^+$ et $NO_3^-$. Il apparaît que, si l'on procède à un traitement dans des conditions réactionnelles douces, le dioxyde cérique est réduit à des dimensions colloïdales par adsorption de l'acide organique, en l'occurrence l'acide oléique, sur les particules de dioxyde cérique ce qui rend aussi les particules colloïdales dispersables en liquides ou solvants organiques non-aqueux. On pense que les dispersions colloïdales obtenues selon le procédé décrit contiennent l'acide solubilisant sous forme d'acide libre et non pas sous forme ionisée. Les produits à base de dioxyde de cérium décrits ci-dessus ne peuvent pas être considérés comme des savons de cérium étant donné que ces savons sont essentiellement des sels de cérium d'acides gras ionisés.

Les exemples qui suivent illustrent l'invention, sans pour autant la limiter.

## Exemple 1

Dans un réacteur à trois cols équipé d'un agitateur, d'un thermomètre et d'un condenseur, on introduit 55 g de $CeO_2$ activée contenant 77,5 % de $CeO_2$ (0,248 mole), 15,5 % d'eau et 7,0 % de nitrate d'ammonium (sous forme d'inclusions), 17,6 g d'acide oléique (0,062 mole) et 35,0 g de solvant AMSCO (hydrocarbures aliphatiques). Le mélange est agité et porté lentement à 90 °C ; on maintient cette température pendant 2 heures tout en agitant. La réaction, mise en évidence par l'apparition de gouttelettes d'eau, démarre entre 55 et 60 °C. Durant ce temps, la suspension solide de $CeO_2$ se transforme en une solution colloïdale de couleur brunâtre contenant de l'eau visible. On ajoute de l'hexane et procède à un séchage azéotropique du système ; on récupère 8,5 g d'eau. On élimine la

majeure partie du composant conduisant à l'azéotrope, puis filtre la solution colloïdale brun foncé.

La teneur en $CeO_2$ est de 44 %.

A titre de comparaison, on agite pendant 4 jours du dioxyde de cérium pur (99,5 % de $CeO_2$) dans du solvant AMSCO en présence d'acide oléique, à une température comprise entre 90 et 140 °C, avec ou sans addition d'eau : il ne se produit aucune dispersion.

Exemples 2 à 21

On réalise les préparations de dioxyde cérique suivantes en procédant comme dans l'exemple 1, en mettant en œuvre le même $CeO_2$ activé, le même rapport molaire du $CeO_2$ à l'acide et au solvant, mais en employant l'acide et le solvant indiqués dans le Tableau I. Dans tous les cas, on obtient une dispersion complète du dioxyde cérique.

Tableau I

| Exemple n° | Solvant | Acide organique | Température de la réaction (°C) | Durée de la réaction (heures) |
|---|---|---|---|---|
| 2 | Toluène | Acide stéarique | 87 | 4 |
| 3 | AMSCO | Acide isostéarique | 103 - 106 | 1 h 30 |
| 4 | AMSCO | Acide laurique + acide oléique | 53 - 117 | 6 |
| 5 | Toluène | Acide dodécylbenzène sulfonique | 90 | 6 |
| 6 | AMSCO | Acide linoléique | 60 - 101 | 45 mn |
| 7 | Dichlorobenzène | Acide oléique | 90 | 4 |
| 8 | AMSCO | Acide myristique | 62 - 118 | 6 |
| 9 | Diisobutylcétone | Acide oléique | 100 | 1 |
| 10 | AMSCO | Acides gras d'huile de coco | 100 | 4 |
| 11 | AMSCO | Acides gras d'huile de soja | 100 | 3 h 30 |
| 12 | p-chlorotoluène | Acide oléique | 100 | 3 |
| 13 | AMSCO | Acide naphténique | 90 | 6 |
| 14 | AMSCO | Acides gras de suif | 85 - 93 | 1 h 30 |
| 15 | Diisobutylcétone | Acide stéarique | 85 - 100 | 4 h 30 |
| 16 | AMSCO | Acides gras d'huile de lin | 70 - 104 | 1 h 30 |
| 17 | AMSCO | Acide caprique | 88 - 115 | 4 h 30 |
| 18 | AMSCO | Acides octoïque* et oléique | 85 - 90 | 1,0 |
| 19 | AMSCO | Acide pélargonique | 85 - 109 | 3 h 30 |
| 20 | Oxyde de mésityle** | Acide oléique | 47 - 90 | une nuit |
| 21 | Ether de dibutyle | Acide oléique | 65 - 95 | une nuit |

* acide éthyl-2 hexoïque

** additionné d'AMSCO pour obtenir une solution limpide

Exemple 22

En suivant le procédé de l'exemple 1 et en utilisant le même solvant et le même acide oléique, on réalise un essai de dispersion du $CeO_2$ activé contenant :

80,17 % de $CeO_2$,

2,9 % de $NH_4NO_3$,

16,9 % de $H_2O$,

séché sous vide à 95 °C et contenant 3,1 g d'eau résiduelle par mole de $CeO_2$ ; on n'a obtenu ni réaction ni dispersion. Ensuite, on ajoute 12 g de méthanol par mole de $CeO_2$ et on soumet le mélange comme

dans l'exemple 1, à un mûrissement, pendant 4 heures à une température allant de 76 à 113 °C. On obtient une dispersion complète. On élimine l'eau et le méthanol ; il en résulte une solution ou dispersion colloïdale dans l'AMSCO contenant 45 % de dioxyde cérique.

Exemples 23 à 25

On chauffe du $CeO_2$ activé ayant la même composition que dans l'exemple 22, séché en étuve à 100 °C pendant 16 heures, mais contenant encore une quantité d'eau résiduelle s'élevant à 6,6 g/mole de $CeO_2$, avec 0,25 mole d'acide oléique par mole de $CeO_2$ avec ou sans addition d'eau et dans les conditions précisées dans le tableau II ci-dessous.

Tableau II

| Exemple n° | Solvant | Quantité totale d'eau g/mole de $CeO_2$ | Température de la réaction (°C) | Durée de la réaction (heures) |
|---|---|---|---|---|
| 23 | Toluène | 22 | 79 - 104 | 3 h 30 |
| 24 | AMSCO | 19,6 | 67 - 109 | une nuit (15 h.) |
| 25 | AMSCO | 6,6 | 50 - 140 | 48 |

Dans les exemples 23 et 24, la dispersion est complète alors qu'elle est incomplète dans l'exemple 25.
L'exemple 25 montre donc que la quantité de 6,6 g d'eau par mole de $CeO_2$ est une valeur limite puisque la dispersion n'est pas complète.

Exemples 26 à 28

On sèche en étuve à 175 °C pendant 72 heures, du $CeO_2$ activé contenant 75 % de $CeO_2$, 6,5 % de $NH_4NO_3$, 16,9 % de $H_2O$. Comme acide organique, on utilise de l'acide oléique à raison de 0,25 mole/mole de $CeO_2$ dans les conditions de l'exemple 26.
Ensuite, on procède au mûrissement du dioxyde de cérium déshydraté en ajoutant de l'eau (exemple 27) ou de l'éthanol (exemple 28) dans les quantités, pendant la durée et aux températures indiquées dans le Tableau III.

Tableau III

| Exemple n° | Solvant | Addition | g/mole de $CeO_2$ | Température (°C) | Durée (heures) |
|---|---|---|---|---|---|
| 26 | Toluène | aucune | - | 90 - 110 | 48 |
| 27 | AMSCO | eau | 33 | 55 - 90 | 4 |
| 28 | Toluène | éthanol | 24 | 90 - 110 | 48 |

Seul l'exemple 27 conduit comme on le désire à une solution ou dispersion colloïdale complète. L'exemple 26 ne contient pas d'eau et l'exemple 28 contient de l'éthanol, mais ni eau, ni méthanol ; aucun des deux ne donne une dispersion apparente de dioxyde cérique.

Exemples 29 à 30

Le $CeO_2$ activé des exemples 26 à 28 précédents est soumis à un séchage azéotropique avec du toluène. Ensuite on ajoute du méthanol (exemple 29) ou de l'acide acétique (exemple 30) avec de l'acide oléique et puis, on disperse la poudre sèche dans les conditions indiquées dans le Tableau IV.

Tableau IV

| Exemple n° | Solvant | Acide | Addition | g/mole de $CeO_2$ | Température de la réaction (°C) | Durée de la réaction |
|---|---|---|---|---|---|---|
| 29 | Toluène | oléïque | MeOH | 10,0 | 74 - 88 | une nuit |
| 30 | Toluène | oléïque | Acide acétique | 18,0 | 67 - 102 | une nuit |

Exemple 31

On chauffe le $CeO_2$ activé de l'exemple 1 à 350 °C pendant 24 heures. Ensuite on mélange le dioxyde cérique avec de l'AMSCO, de l'acide oléique et de l'eau et on procède à un mûrissement comme dans l'exemple 27. Après avoir chauffé pendant plusieurs jours et même après avoir ajouté du méthanol ou de l'acide acétique au mélange réactionnel, on n'observe aucune dispersion.

Exemple 32

On verse lentement un échantillon de 20 g de la solution colloïdale, préparée dans l'exemple 1, dans 100 g d'acétone à température ambiante. Après avoir agité pendant plusieurs heures, à température ambiante, on filtre la matière solide et on la lave plusieurs fois à l'acétone. La matière solide séchée sous vide présente à l'analyse, la composition suivante :

57,9 % de Ce
21,11 % de C
3,48 % de $H_2$
16,51 % de $O_2$ par différence
Formule empirique :
$Ce_4C_{18}H_{38}O_{10}$
Formule chimique :
$(CeO_2)_4$ (acide oléique)

La matière solide obtenue comme précédemment décrit est dispersable dans les hydrocarbures inertes aliphatiques ou cycloaliphatiques, les cétones, les éthers et les solvants hydrocarbonés aromatiques chlorés. La figure 2 correspond à la photographie de ce produit prise au microscope électronique en transmission. Bien que l'acide soit fortement adsorbé à la surface du dioxyde cérique, la simple extraction du méthanol permet de recueillir de l'acide libre en une quantité représentant environ 10 % de la quantité d'acide théoriquement adsorbée.

Cela laisse penser que l'acide oléique est adsorbé à la surface des particules de dioxyde obtenues au cours du processus de dispersion. Cette hypothèse est étayée par l'examen des spectres I.R. des produits de départ et des produits finals. On a déterminé que la vibration d'élongation du groupement carbonyle de l'acide oléique libre était de 1 708 $cm^{-1}$ et cette valeur se situe exactement dans l'intervalle prévu pour un groupement carbonyle relié à une chaîne aliphatique. On n'a pu mettre en évidence la présence d'acide oléique libre dans les spectres I.R. de la matière solide pâteuse ou de la dispersion initiale. Cependant, on a observé une bande forte mais large à 1 510 $cm^{-1}$ (qui n'est pas due au solvant) et l'on attribue cette caractéristique à la vibration d'élongation perturbée du groupement carbonyle de l'acide oléique adsorbé.

Exemple 33

On agite du dioxyde cérique hydraté technique contenant 8,36 % de nitrate d'ammonium et 21,34 % d'eau, au total, avec de l'acide oléique dans du solvant AMSCO à 90 °C comme dans l'exemple 1. La dispersion est réalisée en 17 heures.

On mélange un échantillon de 62,15 g du même dioxyde cérique hydraté avec 14,4 g d'eau et l'on chauffe à une température comprise entre 90 et 100 °C pendant 5 heures. On élimine l'excès d'eau, par filtration ; on ajoute 17,59 g d'acide oléique et 34,95 g de solvant AMSCO et l'on chauffe le mélange à 90 °C. La dispersion est obtenue en 4 heures au lieu de 17.

On chauffe à 400 °C pendant 16 heures, un échantillon de 100 g de dioxyde cérique hydraté utilisé

dans l'exemple 33 ; on obtient 71,8 g d'une poudre sèche contenant 70,3 g de dioxyde de cérium et 1,5 g de nitrates.

Après avoir été chauffé pendant 24 heures à 90 °C, le mélange de 43,9 g de produit sec avec du solvant AMSCO et de l'acide oléique, avec ou sans eau ne donne aucune dispersion.

Exemple 34

Dans du solvant AMSCO, on chauffe, pendant 6 heures seulement, à 90 °C un échantillon de $CeO_2$ activé tel qu'utilisé dans l'exemple 4, puis l'on ajoute l'acide oléique. On obtient une dispersion totale, une heure et demie après l'addition dudit acide.

**Revendications**

1. Procédé de préparation de dispersions colloïdales de dioxyde cérique dans les liquides organiques inertes, caractérisé en ce qu'il consiste :

1) à chauffer à une température comprise dans un intervalle allant d'environ 60 °C à environ 200 °C

a) du dioxyde cérique comprenant du nitrate d'ammonium en une quantité comprise entre environ 3 % et environ 14 % du poids du dioxyde cérique en présence d'eau, de méthanol, d'acide acétique ou de leurs mélanges en une quantité d'au moins 10 g par mole de $CeO_2$

b) un acide organique ayant environ 10 à environ 40 atomes de carbone soluble dans le liquide organique et mis en œuvre à raison d'au moins 0,25 mole par mole de $CeO_2$

c) un liquide organique choisi dans le groupe formé par les hydrocarbures aliphatiques, cycloaliphatiques et aromatiques ; les hydrocarbures chlorés ; les éthers aliphatiques et cycloaliphatiques ; les cétones aliphatiques et cycloaliphatiques formant ainsi une dispersion colloïdale dans le liquide organique du dioxyde cérique et de l'acide organique associé ;

2) à éliminer l'eau, le méthanol, l'acide acétique libéré pendant le chauffage et à séparer toutes les particules solides non dissoutes.

2. Procédé selon la revendication 1 caractérisé par le fait que le dioxyde cérique est le dioxyde cérique, l'hydroxyde cérique, le dioxyde cérique hydraté quelle que soit la forme sous laquelle l'eau est retenue dans ou sur le produit : eau libre, eau liée ou adsorbée, eau de cristallisation.

3. Procédé selon la revendication 1 caractérisé en ce que l'on utilise l'eau, le méthanol ou l'acide acétique ou un mélange d'eau et de méthanol, d'eau et d'acide acétique ou d'eau, de méthanol et d'acide acétique.

4. Procédé selon la revendication 1 caractérisé en ce que le liquide organique est l'hexane, l'heptane, l'octane, le nonane, le décane, le cyclohexane, le cyclopentane, le cycloheptane, les naphtènes liquides, les essences minérales ou de pétrole, éthers minéraux ou de pétrole ; le benzène, le toluène, les xylènes : le choro- ou dichlorobenzène et le chlorotoluène ; l'éther de diisopropyle, l'éther de dibutyle, la diisobutylcétone, l'oxyde de mésityle.

5. Procédé selon la revendication 1 caractérisé en ce que l'acide organique est un acide ou un mélange d'acides naturels ou synthétiques tels que les acides carboxyliques aliphatiques, les acides sulfoniques aliphatiques, les acides phosphoniques aliphatiques, les acides alcoylarylsulfoniques, les acides alcoylarylphosphoniques.

6. Procédé selon la revendication 5 caractérisé en ce que l'acide organique est un acide ou un mélange d'acides tels que les acides gras de tallöl, d'huile de coco, de soja, de suif, d'huile de lin, l'acide oléique, l'acide linoléique, l'acide stéarique, l'acide isostéarique, l'acide pelargonique, l'acide caprique, l'acide laurique, l'acide myristique, l'acide dodécylbenzènesulfonique, l'acide éthyl-2 hexoïque, l'acide naphténique, l'acide hexoïque, l'acide toluène-sulfonique, l'acide toluène-phosphonique, l'acide lauryl-sulfonique, l'acide lauryl-phosphonique, l'acide palmityl-sulfonique et l'acide palmityl-phosphonique.

7. Procédé selon la revendication 6 caractérisé en ce que l'acide organique est un acide ou un mélange d'acides tels qu'un acide oléique, laurique, isostéarique, dodécyl-benzènesulfonique, des acides gras de suif ou d'huile de lin.

8. Procédé selon la revendication 1 caractérisé en ce que la quantité d'eau, de méthanol ou d'acide acétique varie dans l'intervalle de 10 à environ 60 g par mole de $CeO_2$.

9. Procédé selon la revendication 1 caractérisé en ce que l'on chauffe le dioxyde cérique contenant le nitrate d'ammonium en présence d'eau et du liquide organique à une température variant dans l'intervalle d'environ 60° à environ 200 °C et puis on ajoute l'acide organique au mélange résultant.

10. Procédé selon la revendication 9 caractérisé en ce que le dioxyde cérique est sous la forme d'une bouillie aqueuse ou d'une bouillie dans un liquide organique.

11. Procédé selon la revendication 10 caractérisé en ce que le liquide organique est un hydrocarbure aliphatique.

12. Procédé selon la revendication 10 caractérisé en ce que l'acide organique est l'acide oléique.

13. Nouveau complexe associatif de dimensions colloïdales comprenant du dioxyde cérique et un acide organique ayant environ de 10 à environ 40 atomes de carbone dans le rapport molaire $CeO_2$/acide organique de 4/1 et susceptible de se disperser dans le liquide organique défini dans la revendication 1.

14. Complexe selon la revendication 13 caractérisé en ce que l'acide est un acide carboxylique aliphatique, un acide sulfonique aliphatique, un acide phosphonique aliphatique, un acide alcoylarylsulfonique, un acide alcoylarylphosphonique ou leur mélange.

15. Complexe selon la revendication 14 caractérisé en ce que l'acide est un acide gras de tallöl, d'huile de coco, de soja, de suif, d'huile de lin, l'acide oléique, l'acide linoléique, l'acide stéarique, l'acide isostéarique, l'acide pelargonique, l'acide caprique, l'acide laurique, l'acide myristique, l'acide dodécylbenzènesulfonique, l'acide éthyl-2 hexoïque, l'acide naphténique, l'acide hexoïque, l'acide toluènesulfonique, l'acide toluène-phosphonique, l'acide lauryl-sulfonique, l'acide lauryl-phosphonique, l'acide palmityl-sulfonique et l'acide palmitylphosphonique ou leur mélange.

16. Complexe selon la revendication 15 caractérisé en ce que l'acide est un acide oléique, laurique, isostéarique, dodécylbenzène sulfonique, des acides gras de suif ou d'huile de lin ou leur mélange.

17. Dispersion colloïdale comprenant le complexe associatif décrit dans l'une des revendications 13 à 16 dispersé dans un solvant organique.

18. Dispersion selon la revendication 17 caractérisé en ce que le solvant est un liquide organique choisi dans le groupe formé par les hydrocarbures aliphatiques, cycloaliphatiques et aromatiques ; les hydrocarbures chlorés ; les éthers aliphatiques et cycloaliphatiques ; les cétones aliphatiques et cycloaliphatiques.

19. Dispersion selon la revendication 18 caractérisé en ce que le liquide organique est l'hexane, l'heptane, l'octane, le nonane, le décane, le cyclohexane, le cyclopentane, le cycloheptane, les naphtènes liquides, les essences minérales ou de pétrole, éthers minéraux ou de pétrole ; le benzène, le toluène, les xylènes : le choro- ou dichlorobenzène et le chlorotoluène ; l'éther de diisopropyle, l'éther de dibutyle, la diisobutylcétone, l'oxyde de mésityle.

## Claims

1. Process for the preparation of colloidal dispersions of ceric dioxide in inert organic liquids, characterized in that it consists :

1) in heating to a temperature included in a range from approximately 60 °C to approximately 200 °C

a) ceric dioxide containing ammonium nitrate in a quantity of between approximately 3 % and approximately 14 % by weight of the ceric dioxide in the presence of water, methanol, acetic acid or mixtures thereof in a quantity of at least 10 g per mole of $CeO_2$

b) an organic acid containing approximately 10 to approximately 40 carbon atoms, which is soluble in the organic liquid and used in a proportion of at least 0.25 mole per mole of $CeO_2$

c) an organic liquid chosen from the group consisting of aliphatic, alicyclic and aromatic hydrocarbons, chlorinated hydrocarbons, aliphatic and alicyclic ethers, aliphatic and alicyclic ketones thus forming a colloidal dispersion of ceric dioxide and of the associated organic acid in the organic liquid,

2) in removing the water, methanol or acetic acid released during the heating and in separating off all the undissolved solid particles.

2. Process according to Claim 1, characterized in that the ceric dioxide is ceric dioxide, ceric hydroxide, ceric dioxide hydrate, whatever the form in which water is retained in or on the product : free water, bound or adsorbed water, water of crystallization.

3. Process according to Claim 1, characterized in that use is made of water, methanol or acetic acid or a mixture of water and methanol, of water and acetic acid, or of water, methanol and acetic acid.

4. Process according to Claim 1, characterized in that the organic liquid is hexane, heptane, octane, nonane, decane, cyclohexane, cyclopentane, cycloheptane, liquid naphthenes, mineral or petroleum spirits, mineral or petroleum ethers, benzene, toluene, the xylenes, chloro- or dichlorobenzene and chlorotoluene, diisopropyl ether, dibutyl ether, diisobutyl ketone or mesityl oxide.

5. Process according to Claim 1, characterized in that the organic acid is an acid or a mixture of natural or synthetic acids such as aliphatic carboxylic acids, aliphatic sulphonic acids, aliphatic phosphonic acids, alkylarylsulphonic acids or alkylarylphosphonic acids.

6. Process according to Claim 5, characterized in that the organic acid is an acid or a mixture of acids such as the fatty acids from tall oil, coconut, soya, tallow oil, linseed oil, oleic acid, linoleic acid, stearic acid, isostearic acid, pelargonic acid, capric acid, lauric acid, myristic acid, dodecylbenzenesulphonic acid, 2-ethylhexoic acid, naphthenic acid, hexoic acid, toluenesulphonic acid, toluenephosphonic acid, laurylsulphonic acid, laurylphosphonic acid, palmitylsulphonic acid and palmitylphosphonic acid.

7. Process according to Claim 6, characterized in that the organic acid is an acid or a mixture of acids such as oleic, lauric, isostearic, dodecylbenzenesulphonic acid, or fatty acids from tallow or linseed oil.

8. Process according to Claim 1, characterized in that the quantity of water, methanol or acetic acid varies in the range from 10 to approximately 60 g per mole of $CeO_2$.

9. Process according to Claim 1, characterized in that ceric dioxide containing ammonium nitrate is heated in the presence of water and of the organic liquid to a temperature varying in the range from approximately 60° to approximately 200 °C and then the organic acid is added to the resulting mixture.

10. Process according to Claim 9, characterized in that ceric dioxide is in the form of an aqueous slurry or of a slurry in an organic liquid.

11. Process according to Claim 10, characterized in that the organic liquid is an aliphatic hydrocarbon.

12. Process according to Claim 10, characterized in that the organic acid is oleic acid.

13. New association complex of colloidal size comprising ceric dioxide and an organic acid containing approximately from 10 to approximately 40 carbon atoms in the molar ratio $CeO_2$/organic acid of 4/1 and capable of being dispersed in the organic liquid specified in Claim 1.

14. Complex according to Claim 13, characterized in that the acid is an aliphatic carboxylic acid, an aliphatic sulphonic acid, an aliphatic phosphonic acid, an alkylarylsulphonic acid, an alkylarylphosphonic acid or a mixture thereof.

15. Complex according to Claim 14, characterized in that the acid is a fatty acid from tall oil, coconut, soya or tallow oil, linseed oil, oleic acid, linoleic acid, stearic acid, isostearic acid, pelargonic acid, capric acid, lauric acid, myristic acid, dodecylbenzene-sulphonic acid, 2-ethylhexoic acid, naphthenic acid, hexoic acid, toluenesulphonic acid, toluenephosphonic acid, laurylsulphonic acid, laurylphosphonic acid, palmitylsulphonic acid and palmitylphosphonic acid or a mixture thereof.

16. Complex according to Claim 15, characterized in that the acid is an oleic, lauric, isostearic, or dodecylbenzenesulphonic acid, fatty acids from tallow or from linseed oil or a mixture thereof.

17. Colloidal dispersion comprising the association complex described in one of Claims 13 to 16, dispersed in an organic solvent.

18. Dispersion according to Claim 17, characterized in that the solvent is an organic liquid chosen from the group consisting of aliphatic, alicyclic and aromatic hydrocarbons, chlorinated hydrocarbons, aliphatic and alicyclic ethers, and aliphatic and alicyclic ketones.

19. Dispersion according to Claim 18, characterized in that the organic liquid is hexane, heptane, octane, nonane, decane, cyclohexane, cyclopentane, cycloheptane, liquid naphthenes, mineral or petroleum spirits, mineral or petroleum ethers, benzene, toluene, the xylenes, chloro- or dichlorobenzene and chlorotoluene, diisopropyl ether, dibutyl ether, diisobutyl ketone and mesityl oxide.

## Patentansprüche

1. Verfahren zur Herstellung von kolloidalen Dispersionen von Cerdioxid in inerten organischen Flüssigkeiten, dadurch gekennzeichnet, daß es umfasst :

1) Erwärmung auf eine Temperatur im Bereich zwischen etwa 60 °C und 200 °C

a) des Cerdioxids, das Ammoniumnitrat in einer Menge zwischen etwa 3 Gew.% und etwa 14 Gew.% des Cerdioxids enthält, in Gegenwart von Wasser, Methanol, Essigsäure oder ihren Mischungen in einer Menge von mindestens 10 g/Mol $CeO_2$

b) einer organischen Säure mit 10 bis 40 Kohlenstoffatomen, die in der organischen Flüssigkeit löslich ist und in einer Menge von mindestens 0,25 Mol/Mol $CeO_2$ eingesetzt wird,

c) einer organischen Flüssigkeit, die aus der Gruppe der aliphatischen, cycloaliphatischen und aromatischen Kohlenwasserstoffe, der chlorierten Kohlenwasserstoffe, der aliphatischen und cycloaliphatischen Ether und der aliphatischen und cycloaliphatischen Ketone ausgewählt ist, wobei eine kolloidale Dispersion des Cerdioxids und der assoziierten organischen Säure in der organischen Flüssigkeit gebildet wird ;

2) Entfernung des Wassers, Methanols und der während der Erwärmung freigesetzten Essigsäure und Abtrennung aller festen, nicht gelösten Partikel.

2. Verfahren nach Anspruch 1, dadurch gekennzeichnet, daß das Cerdioxid das Cerdioxid, Cer(IV)-hydroxid und hydratisiertes Cer(IV)dioxid ist, unabhängig davon, in welcher Form das Wasser im oder auf dem Produkt vorliegt, ob als freies, gebundenes, adsorbiertes oder Kristallisationswasser.

3. Verfahren nach Anspruch 1, dadurch gekennzeichnet, daß man das Wasser, das Methanol oder die Essigsäure oder ein Gemisch von Wasser und Methanol, von Wasser und Essigsäure oder von Wasser, Methanol und Essigsäure einsetzt.

4. Verfahren nach Anspruch 1, dadurch gekennzeichnet, daß die organische Flüssigkeit Hexan, Heptan, Octan, Nonan, Decan, Cyclohexan, Cyclopentan, Cycloheptan, die flüssigen Naphthene, die Mineral- oder petrostämmigen Öle, mineralische oder Erdölether, Benzol, Toluol, die Xylole, Chlor- oder Dichlorbenzol und Chlortoluol, Diisopropylether, Dibutylether, Diisobutylketon und Mesityloxid sein kann.

5. Verfahren nach Anspruch 1, dadurch gekennzeichnet, daß die organische Säure eine Säure oder ein Gemisch von natürlichen oder synthetischen Säuren wie die aliphatischen Carboxylsäuren, die aliphatischen Sulfonsäuren, die aliphatischen Phosphonsäuren, die Alkylarylsulfonsäuren und die Alkylarylphosphonsäuren sein kann.

6. Verfahren nach Anspruch 5, dadurch gekennzeichnet, daß die organische Säure eine Säure oder ein Gemisch von Säuren ist, wie : Fettsäuren von Tallöl, Kokosöl, Soja, Talg, Leinöl, Oleinsäure, Linolinsäure, Stearin-, Isostearin-, Pelargon-, Caprin-, Laurin-, Myristin-, Dodecylbenzolsulfonsäure, 2-Ethylhexansäure, Naphthen-, Hexan-, Toluolsulfon-, Toluolphosphon-, Laurylsulfon-, Laurylphosphon-, Palmitylsulfon- und Palmitylphosphonsäure.

7. Verfahren nach Anspruch 6, dadurch gekennzeichnet, daß die organische Säure eine Säure oder ein Gemisch von Säuren ist, wie : Olein-, Laurin-, Isostearin-, Dodecylbenzolsäure, Fettsäuren von Talg oder Leinöl.

8. Verfahren nach Anspruch 1, dadurch gekennzeichnet, daß die Menge an Wasser, Methanol oder Essigsäure zwischen 10 und etwa 60 g/Mol $CeO_2$ variiert.

9. Verfahren nach Anspruch 1, dadurch gekennzeichnet, daß man das ammoniumnitrathaltige Cerdioxid in Gegenwart von Wasser und organischer Flüssigkeit auf eine Temperatur im Bereich zwischen etwa 60 °C und etwa 200 °C erwärmt und dann der erhaltenen Mischung die organische Säure zusetzt.

10. Verfahren nach Anspruch 9, dadurch gekennzeichnet, daß das Cerdioxid in Form einer wässrigen Schlempe oder einer Schlempe in einer organischen Flüssigkeit vorliegt.

11. Verfahren nach Anspruch 10, dadurch gekennzeichnet, daß die organische Flüssigkeit ein aliphatischer Kohlenwasserstoff ist.

12. Verfahren nach Anspruch 10, dadurch gekennzeichnet, daß die organische Säure die Oleinsäure ist.

13. Anlagerungskomplex in kolloidalen Dimensionen, der Cerdioxid und eine organische Säure mit zwischen etwa 10 und etwa 40 Kohlenstoffatomen in einem molaren Verhältnis $CeO_2$/organische Säure von 4 : 1 enthält und in der in Anspruch 1 definierten organischen Flüssigkeit dispergiert werden kann.

14. Komplex nach Anspruch 13, dadurch gekennzeichnet, daß die Säure eine aliphatische Carboxyl-, eine aliphatische Sulfon-, eine aliphatische Phosphon-, eine Alkylarylsulfon-, eine Alkylarylphosphonsäure oder eines ihrer Gemische ist.

15. Komplex nach Anspruch 14, dadurch gekennzeichnet, daß die Säure eine Fettsäure von Tallöl, Kokosöl, Soja, Talg, Leinöl, Olein-, Linolin-, Stearin-, Isostearin-, Pelargon-, Caprin-, Laurin-, Myristin-, Dodecylbenzolsulfon-, 2-Ethylhexan-, Naphthen-, Hexan-, Toluolsulfon-, Palmitinsulfon- und Palmitinphosphonsäure oder eines ihrer Gemische ist.

16. Komplex nach Anspruch 15, dadurch gekennzeichnet, daß die Säure, eine Olein-, Laurin-, Isostearin-, Dodecylbenzolsulfonsäure, Fettsäure von Talg oder Leinöl oder eines ihrer Gemische ist.

17. Kolloidale Dispersion, die den Anlagerungskomplex gemäß einem der Ansprüche 13 bis 16 in Dispersion in einem organischen Lösungsmittel enthält.

18. Dispersion nach Anspruch 17, dadurch gekennzeichnet, daß das Lösungsmittel eine organische Flüssigkeit ist, die ausgewählt wird aus der Gruppe der aliphatischen, cycloaliphatischen und aromatischen Kohlenwasserstoffe, der chlorierten Kohlenwasserstoffe, der aliphatischen und cycloaliphatischen Ether, der aliphatischen und cycloaliphatischen Ketone.

19. Dispersion nach Anspruch 18, dadurch gekennzeichnet, daß die organische Flüssigkeit ist : Hexan, Heptan, Octan, Nonan, Decan, Cyclohexan, Cyclopentan, Cycloheptan, flüssige Naphthene, Mineral- oder petrostämmige Öle, mineralische oder Erdölether, Benzol, Toluol, Xylole, Chlor-, oder Dichlorbenzol und Chlortoluol, Diisopropylether, Dibutylether, Diisobutylketon und Mesityloxid.

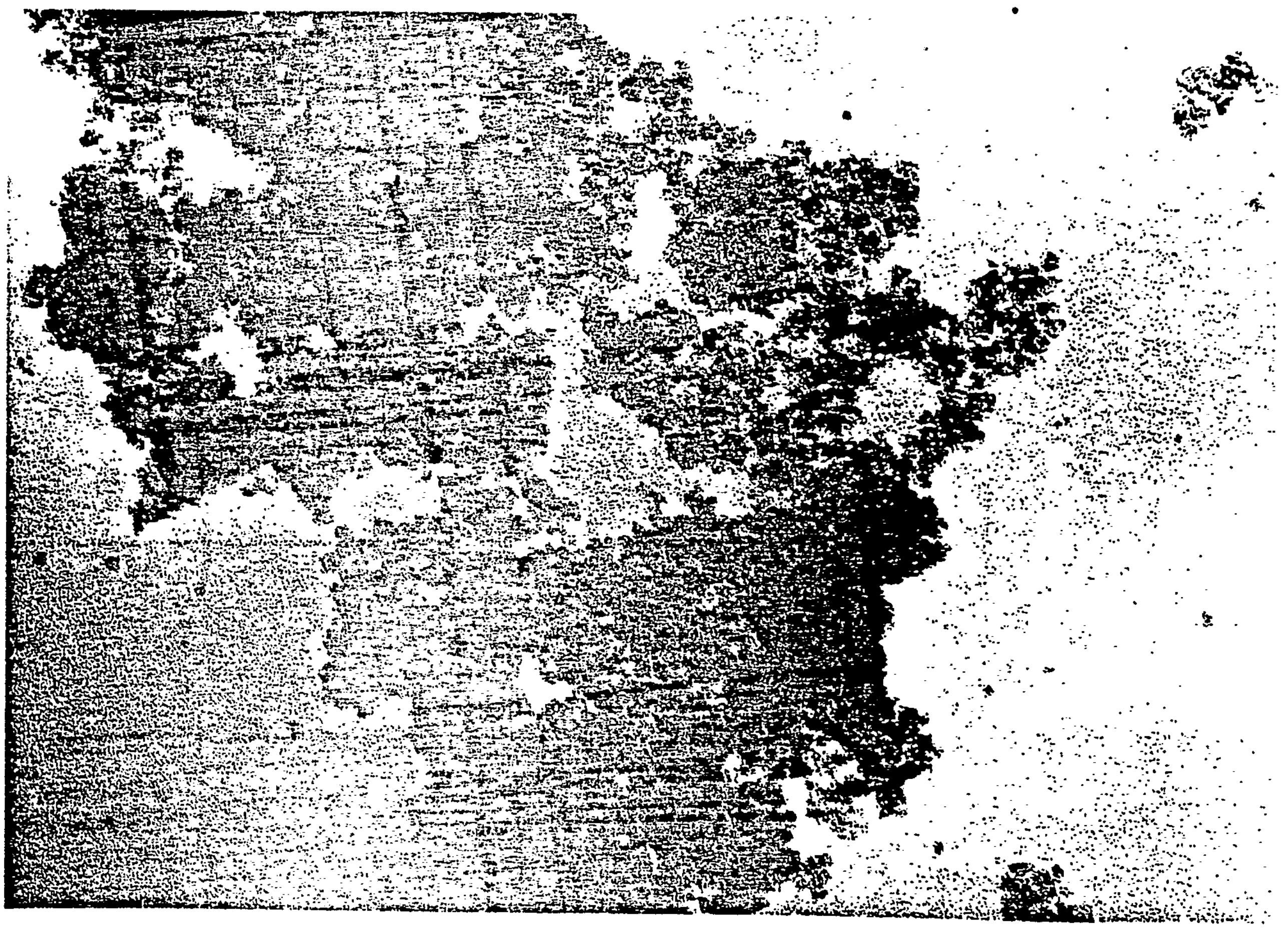

FIG. 1 G = 500.000

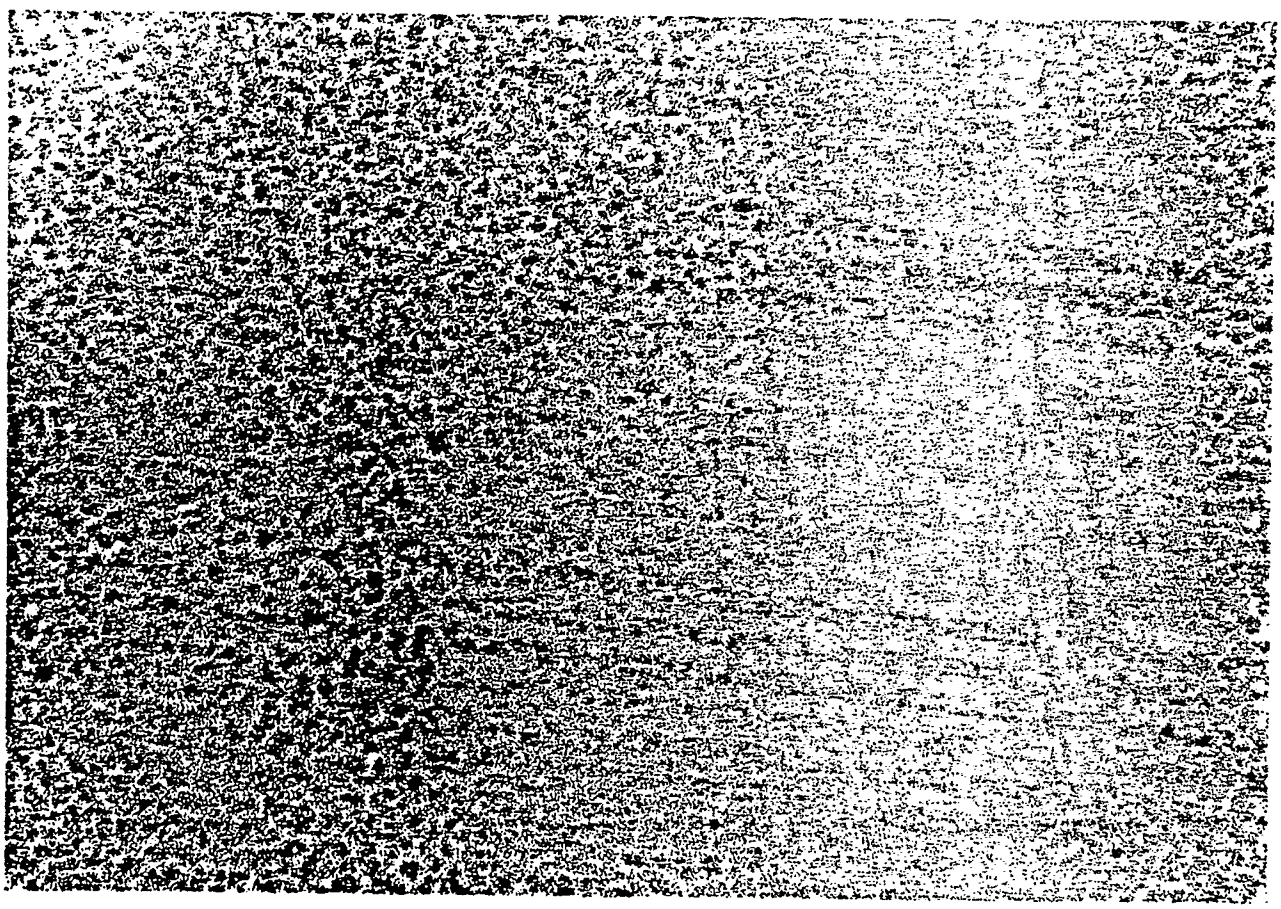

FIG. 2 G = 500.000